# EUROPEAN PATENT APPLICATION

(11) **EP 4 467 216 A1**
(43) Date of publication of application: **27.11.2024**
(21) Application number: 23178428.1
(22) Date of filing: 09.06.2023
(51) Int. Cl.: B01D 3/14, C07D 201/16, B01D 9/00

(54) **A METHOD AND PLANT FOR PURIFYING ETHYLENE CARBONATE**

(30) Priority: 26.05.2023 WO PCT/CN2023/096542
(71) Applicant: Sulzer Management AG, 8401 Winterthur (CH)
(72) Inventor: RIGAUT, Etienne, 68400 Riedisheim (FR); MONDELLI, Cecila, 8057 Zürich (CH); TEMMEL, Erik, 4053 Basel (CH); LI, Yongle, Shanghai (CN); DETTE , Severine, 8408 Winterthur (CH)
(74) Representative: Henkel & Partner mbB

(57) **Abstract**

The present invention relates to a method for purifying ethylene carbonate from a crude ethylene carbonate composition comprising the steps of:
a) subjecting a crude composition containing ethylene carbonate to at least one distillation step so as to obtain a pre-purified composition containing at least 99.5% by weight of ethylene carbonate and
b) subjecting the pre-purified composition obtained in step a) to at least one melt crystallization step so as to obtain a purified ethylene carbonate composition.

## Description

The present invention relates to a method and a plant for purifying ethylene carbonate.

Ethylene carbonate is an important industrial raw material, which is used for instance as solvent or as starting material for the synthesis of ethylene glycols, dimethyl carbonate, ethylene glycol ethers and ethanolamines. Ethylene glycol, in turn, is widely used as chemical, such as for example as antifreeze agent or as monomer in the production of polyester and polyethylene terephthalate, as liquid coolant and as solvent, whereas dimethyl carbonate is used for the manufacture of polycarbonates. Furthermore, ethylene carbonate is gaining more and more importance as electrolyte solvent for lithium-ion batteries, which facilitates the transport of lithium ions from the cathode to the anode. Thereby, ethylene carbonate contributes in a two-fold manner to combat climate change. Firstly, the major route applied in the industrial synthesis of ethylene carbonate is the reaction of ethylene oxide with carbon dioxide, which allows to consume captured carbon dioxide as a feedstock for green chemicals production, and secondly lithium-ion batteries contribute to the decarbonization of the energy sector through electrification, especially in relation to transport. However, considering that high contents of glycols and, especially, of water are detrimental for the long-term performance of batteries, since they promote the formation of hydrogen fluoride, which in turn affects the quality of the solid electrode interface, ethylene carbonate used as electrolyte solvent for lithium-ion batteries needs to be very pure. Common techniques for purifying crude ethylene carbonate are distillation, crystallization, absorption or the like. However, these methods have at least one of the drawbacks of a low separation efficiency such as but not limited to low depletion factors for glycols and/or water, of a high energy demand and of subjecting the ethylene carbonate to some thermal stress leading to its accelerated degradation and formation of by-products and undesired impurities.

In view thereof, the object underlying the present invention is to provide a method and plant for purifying ethylene carbonate being characterized by a particular high productivity, which is the product mass purified per unit of time, by a high recovery yield and by a particular low energy consumption, wherein the method further leads to very pure ethylene carbonate containing 50 ppm or less impurities, such as water and glycols.

In accordance with the present invention, this object is satisfied by providing a method for purifying ethylene carbonate from a crude ethylene carbonate composition comprising the steps of:
a) subjecting a crude composition containing ethylene carbonate to at least one distillation step so as to obtain a pre-purified composition containing at least 99.5% by weight of ethylene carbonate and
b) subjecting the pre-purified composition obtained in step a) to at least one melt crystallization step so as to obtain a purified ethylene carbonate composition.

This solution bases on the finding that by first subjecting a crude ethylene carbonate containing composition, which may for instance originate from a reaction route or may be recovered from any kind of recycling stream, to one or more distillation steps leading to a pre-purified composition containing at least 99.5% by weight of ethylene carbonate, before the pre-purified composition is subjected to one or more melt crystallization steps, not only very pure ethylene carbonate containing at most 50 ppm impurities or even not more than 10 ppm impurities, such as water and glycols, is obtained, but also the method may be performed with a particular low energy consumption, nevertheless with a particular high productivity and with a high recovery yield. This is among others due to the fact that the distillation step(s) may be performed under comparable mild conditions, since the crude ethylene carbonate composition is only purified therein to an ethylene carbonate content of at least 99.5% by weight. This does not only allow to keep the ethylene carbonate recovery rate at more than 90%, but also to maintain the energy consumption at a low level, because more stringent distillation conditions or further distillation steps consuming much more energy for achieving a higher purity degree of ethylene carbonate, such as ethylene carbonate containing 200 to 800 ppm impurities, are not necessary. Such a high purity may be achieved by distillation only with an energy consumption being between 3 and 6 times higher compared to the energy consumption of a distillation step being performed under mild conditions so as to obtain a pre-purified composition containing at least 99.5% by weight of ethylene carbonate. The further purification of the pre-purified composition is then performed in accordance with the present invention in one or more melt crystallization steps, which leads to purified ethylene carbonate containing at most 50 ppm or even at most 10 ppm impurities. It has been found in the present invention that melt crystallization is characterized by a high separation efficiency for ethylene carbonate from water and glycols for each crystallization stage as well as by very high depletion factors of significantly higher than 10 for glycols, if a pre-purified composition containing at least 99.5% by weight of ethylene carbonate is used as starting composition for the melt crystallization. Depletion factor means with this regard the ratio of the concentration of a substance in the feed of a melt crystallization stage divided by the concentration of the product as obtained as the result of the same melt crystallization stage. Even if it would be possible to purify the crude ethylene carbonate composition solely by melt crystallization without any distillation step, this would require a very high number of crystallization stages in order to obtain a purification to an impurity content of at most 50 ppm. Such a high number of crystallization stages would lead to very high operational costs due to a particular high energy consumption. Furthermore, this would require a large size of the crystallization apparatus. Thus, the distillation step(s) a) leading to just a pre-purified composition containing at least 99.5% by weight of ethylene carbonate and the crystallization step(s) b) synergistically work together and lead with a particular high productivity and with a high recovery yield to a very pure ethylene carbonate containing at most 50 ppm impurities or even not more than 10 ppm impurities at a particular low energy consumption. More specifically, the energy savings are 20 to 25% compared to the known methods.

As set out above, step a) is intentionally performed so as to obtain a pre-purified composition containing at least 99.5% by weight of ethylene carbonate, i.e. a composition, which needs to be not so pure to have only low ppm contents of impurities. Thereby, the number of distillation steps may be kept low as well as the temperature and pressure conditions during the distillation step(s) may be kept moderate so that the energy demand for the distillation is quite low. Good results are in particular obtained, when in step a) at least one distillation step is performed so as to obtain a pre-purified composition containing 99.5 to 99.9% by weight of ethylene carbonate. The content of glycol impurities in the pre-purified composition is preferably 100 to 2,000 ppm. More preferably, in step a) at least one distillation step is performed so as to obtain a pre-purified composition containing 99.6 to 99.9% by weight, even more preferably of 99.7 to 99.9% by weight, still more preferably of 99.8 to 99.9% by weight and most preferably of 99.85 to 99.90% by weight of ethylene carbonate.

In a further development of the idea of the present invention, it is suggested that the crude composition used in step a) contains 10 to 99% by weight, preferably 50 to 99% by weight, more preferably 70 to 99% by weight and most preferably 95 to 99% by weight of ethylene carbonate.

As set out above, step a) comprises at least one distillation step. Thus, step a) may comprise two or more distillation steps. However, it is preferred that step a) does not comprise more than two distillation steps. For instance, step a) comprises two subsequent distillation steps. The crude composition may be fed in step a) into a first distillation column and distilled therein into an overhead fraction, a side fraction and a bottom fraction, wherein the bottom fraction is led into a second distillation column and distilled therein into an overhead fraction, a side fraction and a bottom fraction, wherein the overhead fraction is led as the pre-purified composition to the at least one melt crystallization step of step b). However, it is preferred in this embodiment that the crude composition is fed in step a) into a first distillation column and distilled therein into an overhead fraction, into a bottom fraction and into a side fraction, wherein the side fraction is led into a second distillation column and distilled therein into an overhead fraction, into a bottom fraction and into a side fraction, wherein the side fraction is led as the pre-purified composition to the at least one melt crystallization step of step b).

In accordance with a particularly preferred embodiment of the present invention, step a) comprises (only) one distillation step, wherein the crude composition is fed in step a) into a distillation column and is distilled therein preferably into an overhead fraction, into a bottom fraction and into a side fraction, wherein the side fraction is led as the pre-purified composition to the at least one melt crystallization step of step b).

Particularly good results are obtained, when in step a) as the distillation column, if step a) comprises one distillation step, or as the first distillation column, if step a) comprises two or more distillation steps, a bottom dividing-wall distillation column is used, which comprises a dividing wall being connected with and extending from the bottom of the distillation column at least substantially vertically upwards over a part of the height of the distillation column thereby subdividing the bottom section of the dividing-wall distillation column, seen in cross-section of the dividing-wall distillation column, into a first bottom subsection and into a second bottom subsection. Bottom section means in this connection the section of the distillation column extending from the uppermost to the lowermost part of the dividing wall. The first bottom subsection is the subsection of the bottom section being located on the side of the distillation column, into which the inlet for the crude ethylene carbonate composition leads, whereas the second bottom subsection is opposite to the first bottom subsection. Finally, at least substantially vertically means that the angle between the dividing wall and the vertical direction is at most 10°, preferably at most 5°, more preferably at most 1° and most preferably 0°.

In accordance with a further preferred embodiment of the present invention, the dividing wall subdivides the bottom section of the dividing-wall distillation column, seen in cross-section of the dividing-wall distillation column, in about two subsections. Therefore, it is preferred that one of the first and second bottom subsections covers at least 50%, more preferably at least 55%, yet more preferably at least 60% and most preferably 65 to 70% of the total area of the cross-section of the bottom section of the dividing-wall distillation column, whereas the other of the first and second bottom subsections covers the remainder to 100% of the total area of the cross-section of the bottom section of the dividing-wall distillation column. For instance, one subsection covers 43% and the other 57% of the total area of the cross-section or both subsections cover each exactly 50% of the total area of the cross-section. If the total area of the cross-section of the bottom section varies over the axial length of the bottom section, the above numeric values are related to the average total area of the cross-section of the bottom section.

Good results are in particular obtained, when the dividing wall extends in the distillation column from its bottom over 1 to 30% and preferably 5 to 20% of the height of the distillation column.

In accordance with a further particularly preferred embodiment of the present invention, the first bottom subsection of the bottom dividing-wall distillation column comprises an outlet line and the bottom dividing-wall distillation column comprises within or above the first bottom subsection an inlet line, both being connected with a recycle line leading from the outlet line to the inlet line, wherein the recycle line is connected with a first reboiler. Good results are in particular obtained, when the first reboiler is a falling film evaporator.

Likewise thereto, it is also preferred that the second bottom subsection comprises an outlet line and the bottom dividing-wall distillation column comprises within or above the second bottom subsection an inlet line, both being connected with a recycle line leading from the outlet line to the inlet line, wherein the recycle line is connected with a second reboiler, which is operated at an operating pressure that is at least 25%, preferably 40% and even more preferably 50% lower than the first reboiler. Good results are in particular obtained, when the second reboiler is a wiped film evaporator, a falling film evaporator or a short path evaporator.

Preferably, both aforementioned embodiments are combined, i.e. the first bottom subsection comprises an aforementioned recycle circuit with a first reboiler, which is preferably a falling film evaporator, and the second bottom subsection comprises an aforementioned recycle circuit with a second reboiler, which is preferably a wiped film evaporator, a falling film evaporator or a short path evaporator.

In addition to the aforementioned dividing wall, the bottom dividing-wall distillation column preferably further comprises a partition wall preferably comprising a first portion extending at least essentially vertically downwards and a second portion being connected with an upper part of the first portion of the partition wall and extending to the inner wall of the bottom dividing-wall distillation column, wherein the first portion of the partition wall extends above and/or at least partially within the second bottom subsection. The inlet line being connected with the reboiler being connected with the outlet line of the second bottom subsection is preferably connected in this embodiment with the wall of the bottom dividing-wall distillation column at a location being below the second portion of the partition wall and between the upper and lower ends of the first portion of the partition wall. The second portion of the partition wall may extend inclined with regard to the horizontal plane, such as inclined by an angle of more than 10° to 40° with regard to the horizontal plane, or may extend at least substantially horizontal, wherein at least substantially horizontal means that the angle between the second portion of the partition wall and the horizontal plane is at most 10°, preferably at most 5°, more preferably at most 1° and most preferably 0°. The second portion of the partition wall is preferably fluid tightly connected with the inner wall of the bottom dividing-wall distillation column, which means that neither descending liquid nor ascending vapor may cross the second portion of the partition wall. The terms dividing wall and partition wall each denote any kind of wall, wherein the "dividing" and "partition" are only used to easily distinguish between the two walls.

In accordance with a further particularly preferred embodiment of the present invention, the bottom dividing-wall distillation column comprises a side fraction outlet and further preferably comprises a separation wall. Also the term separation wall denotes any kind of wall, wherein the "separation" is only used to easily distinguish this wall from the aforementioned dividing wall and the aforementioned partition wall. The separation wall comprises a first portion extending at least essentially vertically downwards and a second portion being connected with an upper part of the first portion of the separation wall and extending to the inner wall of the bottom dividing-wall distillation column. While the first portion of the separation wall extends above and/or at least partially within the first bottom subsection, the inlet line being connected with the reboiler being connected with the outlet line of the first bottom subsection is connected with the bottom dividing-wall distillation column at a location being below the second portion of the separation wall and between the upper and lower ends of the first portion of the separation wall, and the uppermost point of the second portion of the separation wall is located below the side fraction outlet. The second portion of the partition wall may extend inclined with regard to the horizontal plane, such as inclined by an angle of more than 10° to 40° with regard to the horizontal plane, or may extend at least substantially horizontal, wherein at least substantially horizontal means that the angle between the second portion of the partition wall and the horizontal plane is at most 10°, preferably at most 5°, more preferably at most 1° and most preferably 0°. The second portion of the partition wall is preferably fluid tightly connected with the inner wall of the bottom dividing-wall distillation column, which means that neither descending liquid nor ascending vapor may cross the second portion of the partition wall. The side fraction outlet is located between the top and the bottom and is preferably located, seen from the bottom to the top of the bottom dividing-wall distillation column, at a position being located at 40 to 90% and preferably 60 to 90% of the height of the bottom dividing-wall distillation column.

In a further development of the idea of the present invention, it is preferred that the overhead of the bottom dividing-wall distillation column comprises an outlet line and an inlet line, both being connected with a recycle line leading from the outlet line to the inlet line, wherein the recycle line is connected with an overhead condenser or cold trap, respectively, and downstream thereof with a gas-liquid separator, wherein the gas-liquid separator is connected with a liquid line and is connected with a gas line, wherein the liquid line leads back into the bottom dividing-wall distillation column.

Good results are in particular obtained, when the sidewall of the bottom dividing-wall distillation column comprises an outlet line and an inlet line, both being connected with a recycle line leading from the outlet line to the inlet line, wherein the recycle line is connected with a side condenser and downstream thereof with a gas-liquid separator, wherein the gas-liquid separator is connected with a liquid line and is connected with a gas line, wherein the liquid line leads back into the bottom dividing-wall distillation column.

In order to minimize the energy consumption during the method, it is further proposed to perform the distillation step(s) so as to allow heat integration. For instance, the distillation is performed in the at least one distillation step so that the condensation temperature of the overhead fraction is at least 100°C, preferably 110 to 150°C and more preferably 115 to 125°C. This allows to generate in the overhead condenser a heat transfer medium stream with a temperature of at least 80°C, preferably of 90 to 130°C and more preferably of 95 to 105°C.This heat transfer medium can in turn be used to power another step, such as the sweating and/or melting of the ethylene carbonate crystals obtained in the at least one melt crystallization step, as described further below. This allows to reduce the overall energy consumption by up to 25%, compared to a respective system without implementation of any heat integration.

Furthermore, it is preferred that the distillation column(s) used in step a) comprise(s) one or more internals, such as one or more beds of structured packings, one or more beds of random packings or one or more trays. This leads to a particular efficient mass and heat transfer between the descending liquid phase and the ascending gas phase. More preferably, the distillation column(s) used in step a) comprise(s) one or more beds of structured packings, such as one to five and preferably two to four beds of structured packings. Good results are in particular obtained, when each of the structured packings has a specific surface area 100 to 750 m²/m³ and more preferably of 150 to 500 m²/m³.

The present invention is neither particularly limited concerning the kind of at least one melt crystallization stage nor concerning the number of melt crystallization stages. Good results are in particular obtained, when the at least one melt crystallization step b) comprises at least one melt crystallization stage being selected from the group consisting of falling film crystallization stages, static crystallization stages or suspension crystallization stages. Falling film crystallization means also dynamic crystallization. Each of the aforementioned crystallization techniques may comprise one to five, preferably one to four, more preferably one to three and most preferably one to two melt crystallization stages.

In accordance with a particularly preferred embodiment of the present invention, the at least one melt crystallization step b) comprises at least one falling film crystallization stage. During a falling film crystallization stage, melt to be crystallized flows along a cooled surface downwards, such as along the inside of a cooled tube, which allows crystals to grow from the falling film of melt on the inside surface of the tube, which is cooled by a falling film of a cooling agent co-currently flowing on the outside surface of the tube. High and very reproducible transfer rates are achieved on both side of the tube, wherein the resulting shear at the crystal/liquid interface transports impurities rapidly into the bulk of the melt. Preferably, the falling film crystallizer used for the crystallization stage(s) b) contains a plurality of vertical tubes, in which the crystal layers grow as cylindrical shells, a collection vessel beneath the tubes as well as a circulating pump. Before the start of a crystallization stage, the collection vessel is filled with a batch of the melt to be crystallized. The circulating pump is then started to wet the tubes and initiate crystallization at a certain temperature level, while coolant temperature ramping is started. The melt circulation rate is adjusted to a high value compared to the rate of crystal deposition so that conditions of temperature and composition are approximately uniform across the length of the tubes. The temperature is ramped down at a constant rate until the collecting vessel level drops to a preset value. At this point, melt circulation is discontinued.

Good results are in particular obtained, when step b) comprises one to five, preferably one to four, more preferably one to three and most preferably one to two falling film crystallization stages.

Preferably, the pre-purified composition obtained in step a) and subjected in step b) to at least one melt crystallization step is fed into a first of the two to five falling film crystallization stages so as to produce a first ethylene carbonate enriched crystallized fraction and a residue fraction, wherein the first ethylene carbonate enriched crystallized fraction is fed into a second of the two to five falling film crystallization stages, wherein in any of the second and of the optional third to five falling film crystallization stages an ethylene carbonate enriched crystallized fraction and a residue fraction is produced, wherein each of the ethylene carbonate enriched crystallized fractions produced in the second and the optional third to fourth falling film crystallization stages is fed into a downstream ethylene carbonate crystallization stage and each of the residue fractions produced in the second and the optional third to fifth falling film crystallization stages is fed into an upstream falling film crystallization stage, wherein the ethylene carbonate enriched crystallized fraction obtained in the most downstream of the falling film crystallization stages is the purified ethylene carbonate composition.

In addition, it is preferred that the production of an ethylene carbonate enriched crystallized fraction and of a residue fraction in a crystallization stage comprises the steps of removing the remaining liquid from the crystallization stage as residue fraction after termination of the crystallization in the crystallization stage, of melting the crystal layer obtained in the crystallization stage and of withdrawing the obtained crystal melt as ethylene carbonate enriched crystallized fraction from the crystallization stage.

In a further development of the idea of the present invention, it is suggested that before melting the crystal layer obtained in the crystallization stage, one or more sweating steps of the crystal layer are carried out so as to obtain one or more sweating fractions and a purified crystal layer, wherein preferably at least a portion of the first sweating fraction obtained thereby is fed to the remaining liquid which has been removed as residue fraction. Sweating is achieved by increasing the temperature of the crystals to a numeric value being just below the melting point of ethylene carbonate, such as 0.1 to 2°C below the melting point of ethylene carbonate, in order to liquefy impurities and assist further draining.

Good results are in particular obtained, when at least one and preferably all of the melt crystallization step(s) is/are performed at a temperature of -10°C to 70°C, preferably at a temperature of -5°C to 65°C and more preferably at a temperature of 0°C to 60°C.

In accordance with a particular preferred embodiment of the present invention, the purified ethylene carbonate composition comprises 50 ppm or less and preferably 10 ppm or less impurities.

In a further aspect, the present invention relates to a plant for purifying ethylene carbonate from a crude ethylene carbonate composition, wherein the plant comprises at least one distillation column and at least one crystallizer, wherein the at least one distillation column is a bottom dividing-wall distillation column, which comprises a dividing wall being connected with and extending from the bottom of the distillation column at least substantially vertically upwards over a part of the height of the distillation column thereby subdividing the bottom section of the dividing-wall distillation column, seen in cross-section of the dividing-wall distillation column, into a first bottom subsection and into a second bottom subsection, wherein the bottom dividing-wall distillation column further comprises an inlet line for a crude composition containing ethylene carbonate, an overhead outlet line, a bottom outlet line and a side outlet line, wherein at least one crystallizer comprises an inlet line for a pre-purified composition and an outlet line for a purified ethylene carbonate composition, wherein the side outlet line of the bottom dividing-wall distillation column is connected with the inlet line for a pre-purified composition of the at least one crystallizer.

Good results are in particular obtained, when the dividing wall extends in the distillation column from its bottom over 1 to 30% and preferably 5 to 20% of the height of the distillation column.

Furthermore, it is preferred that the dividing wall subdivides the bottom section of the dividing-wall distillation column, seen in cross-section of the dividing-wall distillation column, into a first bottom subsection covering at least 30%, more preferably at least 40%, yet more preferably at least 45% and most preferably 50% of the total area of the cross-section of the bottom section of the dividing-wall distillation column, whereas the other of the first and second bottom subsections covers the remainder to 100% of the total area of the cross-section of the bottom section of the dividing-wall distillation column.

In a further development of the idea of the present invention, it is proposed that the first bottom subsection of the bottom dividing-wall distillation column comprises an outlet line and the bottom dividing-wall distillation column comprises within or above the first bottom subsection an inlet line, both being connected with a recycle line leading from the outlet line to the inlet line, wherein the recycle line is connected with a first reboiler, and wherein the second bottom subsection comprises an outlet line and the bottom dividing-wall distillation column comprises within or above the second bottom subsection an inlet line, both being connected with a recycle line leading from the outlet line to the inlet line, wherein the recycle line is connected with a second reboiler. Preferably, the first reboiler is a falling film evaporator and the second reboiler is a wiped film evaporator, a falling film evaporator or a short path evaporator and most preferably a wiped film evaporator.

In accordance with a further particular preferred embodiment of the present invention, the bottom dividing-wall distillation column further comprises a partition wall comprising a first portion extending at least essentially vertically downwards and a second portion being connected with an upper part of the first portion of the partition wall and extending to the inner wall of the bottom dividing-wall distillation column, wherein the first portion of the partition wall extends above and/or at least partially within the second bottom subsection, wherein the inlet line being connected with the reboiler being connected with the outlet line of the second bottom subsection is connected with the wall of the bottom dividing-wall distillation column at a location being below the second portion of the partition wall and between the upper and lower ends of the first portion of the partition wall.

In accordance with a further particular preferred embodiment of the present invention, the bottom dividing-wall distillation column comprises a side fraction outlet and further comprises a separation wall comprising a first portion extending at least essentially vertically downwards and a second portion being connected with an upper part of the first portion of the separation wall and extending to the inner wall of the bottom dividing-wall distillation column, wherein the first portion of the separation wall extends above and/or at least partially within the first bottom subsection, wherein the inlet line being connected with the reboiler being connected with the outlet line of the first bottom subsection is connected with the bottom dividing-wall distillation column at a location being below the second portion of the separation wall and between the upper and lower ends of the first portion of the separation wall, wherein the uppermost point of the second portion of the separation wall is located below the side fraction outlet.

Good results are in particular obtained, when the at least one crystallizer comprises one to five, preferably one to four, more preferably one to three and most preferably one to two falling film crystallization stages.

The invention will be explained in more detail hereinafter with reference to the drawings, in which:
- Fig. 1: schematically shows a plant for purifying ethylene carbonate from a crude ethylene carbonate composition in accordance with one embodiment of the present invention.
- Fig. 2: schematically shows a plant for purifying ethylene carbonate from a crude ethylene carbonate composition in accordance with another embodiment of the present invention.
- Fig. 3: schematically shows the bottom portion of an alternative bottom dividing-wall distillation column, which may be used in a plant shown in figures 1 and 2.
- Fig. 4: schematically shows the bottom portion of an alternative distillation column, which may be used in a plant shown in figures 1 and 2.

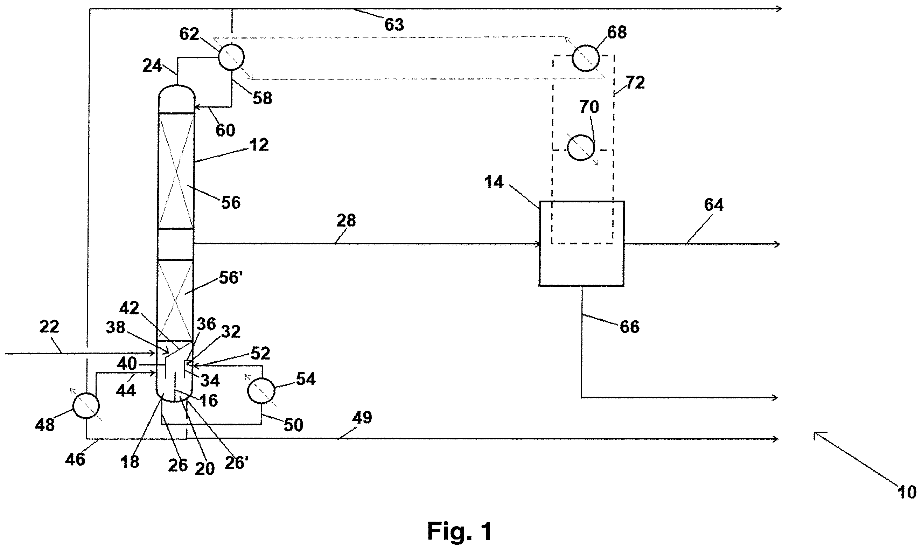

The plant 10 for purifying ethylene carbonate from a crude ethylene carbonate composition shown in figure 1 comprises a distillation column 12 and a crystallizer 14. More specifically, the distillation column 12 is a bottom dividing-wall distillation column 12, which comprises a dividing wall 16 being connected with and extending from the bottom of the distillation column 12 vertically upwards over a part of the height of the distillation column 12, thereby subdividing the bottom section of the dividing-wall distillation column 12, seen in cross-section of the dividing-wall distillation column 12, into a first bottom subsection 18 and into a second bottom subsection 20. The dividing wall 16 extends in the distillation column 12 from its bottom over about 10% of the height of the distillation column 12. Moreover, the bottom dividing-wall distillation column 12 comprises an inlet line 22 for a crude composition containing ethylene carbonate, an overhead outlet line 24, two bottom outlet lines 26, 26' and a side outlet line 28 for pre-purified composition to be led into the crystallizer. In addition, the bottom dividing-wall distillation column 12 comprises a partition wall 32, which comprises a first portion 34 extending vertically downwards and a second portion 36 being connected with the uppermost part of the first portion 34 of the partition wall 32 and extending to the inner wall of the bottom dividing-wall distillation column 12. The first portion 34 of the partition wall 32 extends above and partially within the second bottom subsection 20 of the bottom dividing-wall distillation column 12. Furthermore, the bottom dividing-wall distillation column 12 comprises a separation wall 38, which comprises a first portion 40 extending vertically downwards and a second portion 42 being connected with an upper part of the first portion of the separation wall and extending to the inner wall of the bottom dividing-wall distillation column 12, wherein the first portion 40 of the separation wall 38 extends above and at least partially within the first bottom subsection 18.

The bottom dividing-wall distillation column 12 further comprises an inlet line 44, which is connected with a recycle line 46 leading from the outlet line 26 of the first bottom subsection 18 to the inlet line 44, which leads into the first bottom subsection 18 of the bottom dividing-wall distillation column 12, wherein the recycle line 46 is connected with a first reboiler 48 and with an outlet line 49 for heavies. The first reboiler 48 is preferably a wiped film evaporator. Likewise thereto, also the outlet line 26' of the second bottom subsection 20 is connected with a recycle line 50 leading from the outlet line to an inlet line 52, which leads into the second bottom subsection 20 of the bottom dividing-wall distillation column 12. The recycle line 50 is connected with a second reboiler 54, wherein the second reboiler 54 is preferably a falling film evaporator. Moreover, the bottom dividing-wall distillation column 12 comprises two beds of structured packing 56, 56', one 56 of which being arranged above the side outlet line 28 and the other 56' of which being arranged below the side outlet line 28 and above the separation wall 38. In addition, the outlet line 24 of the overhead of the bottom dividing-wall distillation column 12 is connected with a recycle line 58, which in turn is connected with an inlet line 60 leading into the top portion of the bottom dividing-wall distillation column 12. In turn,, the recycle line 58 is connected with an overhead condenser 62, which is in addition connected with a outlet line 63 for lights.

The crystallizer 14 comprises three falling film crystallization stages, an outlet line 64 for pure ethylene carbonate and an outlet line 66 for residue. Furthermore, the crystallizer 14 is connected with a heater 68 and a cooler 70, which form together with the condenser 62 a heat integration system 72.

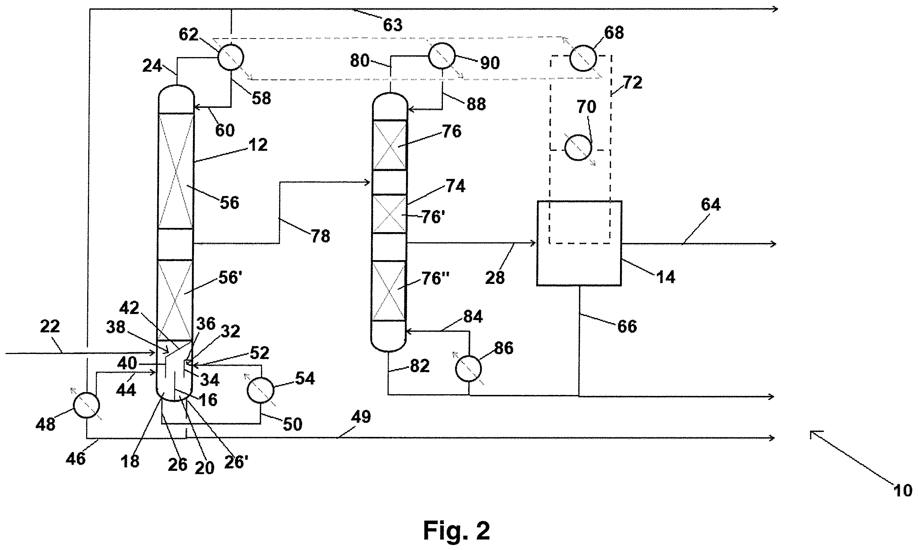

The plant shown in figure 2 correspond to that of figure 1 except that it further comprises a second distillation column 74, which is arranged between the bottom dividing-wall distillation column 12 and the crystallizer 14. More specifically, the second distillation column 74 comprises three beds of structured packings 76, 76', 76", wherein a connection line 78 leads from the bottom dividing-wall distillation column 12 to the second distillation column 74 and enters the second distillation column 74 at a location between the uppermost and middle beds of structured packings 76, 76'. Also the second distillation column 74 comprises an overhead outlet 80, a bottom outlet 82 and further comprises a recycle line 84 being provided with a reboiler 86 and leading from the bottom outlet line 82 back into the bottom portion of the second distillation column 74. Likewise thereto, the second distillation column 74 further comprises a recycle line 88 being provided with a condenser 90 and leading from the overhead outlet line 80 back into the overhead portion of the second distillation column 74.

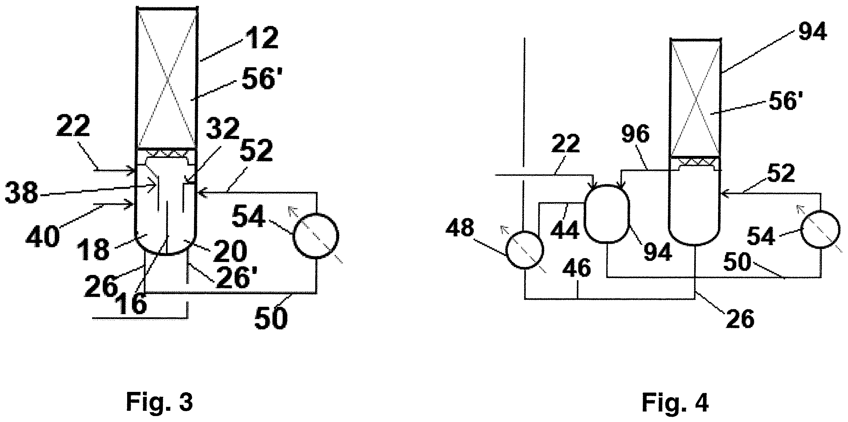

Figure 3 shows an alternative bottom portion of a bottom dividing-wall distillation column 12, which may be used instead of that shown in figures 1 and 2. The bottom dividing-wall distillation column 12 shown in Figure 3 corresponds to that shown in figures 1 and 2 except that the shape of the separation wall is slightly different.

Figure 4 shows an alternative bottom portion of a distillation column 92, which may be used instead of the bottom dividing-wall distillation 12 shown in figures 1 and 2. The distillation column 92 does not contain a dividing wall, does not contain a partition wall and does not contain a separation wall. However, it comprises a vessel 94, to which a line 96 leads and into which also the inlet line 22 for a crude composition containing ethylene carbonate leads.

### Reference Numerals

- 10: Plant
- 12: (Bottom dividing-wall) distillation column
- 14: Crystallizer
- 16: Dividing wall
- 18: First bottom subsection of the bottom dividing-wall distillation column
- 20: Second bottom subsection of the bottom dividing-wall distillation column
- 22: Inlet line of the bottom dividing-wall distillation column
- 24: Overhead outlet line of the bottom dividing-wall distillation column
- 26, 26': Bottom outlet lines of the bottom dividing-wall distillation column
- 28: Side outlet line for pre-purified composition
- 32: Partition wall
- 34: First portion of the partition wall
- 36: Second portion of the partition wall
- 38: Separation wall
- 40: First portion of the separation wall
- 42: Second portion of the separation wall
- 44: Inlet line
- 46: Recycle line
- 48: First reboiler
- 49: Outlet line for heavies
- 50: Recycle line
- 52: Inlet line
- 54: Second reboiler
- 56, 56': Bed of structured packing
- 58: Recycle line
- 60: Inlet line
- 62: Overhead condenser
- 63: Outlet line for lights
- 64: Outlet line for pure ethylene carbonate
- 66: Outlet line for resuidue
- 68: Heater
- 70: Cooler
- 72: Heat integration system
- 74: Second distillation column
- 76, 76', 76": Bed of structured packing of second distillation column
- 78: Connection line
- 80: Overhead outlet line of the second distillation column
- 82: Bottom outlet lines of the second distillation column
- 84: Recycle line
- 86: Reboiler
- 88: Recycle line
- 90: Condenser
- 92: Distillation column
- 94: Vessel
- 96: Line

## Claims

1. A method for purifying ethylene carbonate from a crude ethylene carbonate compositions comprising the steps of:
a) subjecting a crude composition containing ethylene carbonate to at least one distillation step so as to obtain a pre-purified composition containing at least 99.5% by weight of ethylene carbonate and
b) subjecting the pre-purified composition obtained in step a) to at least one melt crystallization step so as to obtain a purified ethylene carbonate composition.

2. The method in accordance with claim 1, wherein the crude composition is fed in step a) into a first distillation column and distilled therein into an overhead fraction, into a bottom fraction and into a side fraction, wherein the side fraction is led into a second distillation column and distilled therein into an overhead fraction, into a bottom fraction and into a side fraction, wherein the side fraction is led as the pre-purified composition to the at least one melt crystallization step of step b)

3. The method in accordance with claim 1 or 2, wherein in step a) as the distillation column, if step a) comprises one distillation step, or the first distillation column, if step a) comprises two or more distillation steps, a bottom dividing-wall distillation column is used, which comprises a dividing wall being connected with and extending from the bottom of the distillation column at least substantially vertically upwards over a part of the height of the distillation column thereby subdividing the bottom section of the dividing-wall distillation column, seen in cross-section of the dividing-wall distillation column, into a first bottom subsection and into a second bottom subsection, wherein at least substantially vertically means that the angle between the at least one dividing wall and the vertical direction is at most 10°, preferably at most 5°, more preferably at most 1° and most preferably 0°.

4. The method in accordance with claim 3, wherein the dividing wall extends in the distillation column from its bottom over 1 to 30% and preferably 5 to 20% of the height of the distillation column, and, wherein the dividing wall subdivides the bottom section of the dividing-wall distillation column, seen in cross-section of the dividing-wall distillation column, into a first bottom subsection covering at least 50%, more preferably at least 55%, yet more preferably at least 60% and most preferably 65 to 70% of the total area of the cross-section of the bottom section of the dividing-wall distillation column, whereas the other of the first and second bottom subsections covers the remainder to 100% of the total area of the cross-section of the bottom section of the dividing-wall distillation column.

5. The method in accordance with claim 3 or 4, wherein the first bottom subsection of the bottom dividing-wall distillation column comprises an outlet line and the bottom dividing-wall distillation column comprises within or above the first bottom subsection an inlet line, both being connected with a recycle line leading from the outlet line to the inlet line, wherein the recycle line is connected with a first reboiler, and wherein the second bottom subsection comprises an outlet line and the bottom dividing-wall distillation column comprises within or above the second bottom subsection an inlet line, both being connected with a recycle line leading from the outlet line to the inlet line, wherein the recycle line is connected with a second reboiler, wherein the first reboiler is preferably a falling film evaporator and the second reboiler is a wiped film evaporator, a falling film evaporator or a short path evaporator.

6. The method in accordance with any of claims 3 to 5, wherein the overhead of the bottom dividing-wall distillation column comprises an outlet line and an inlet line, both being connected with a recycle line leading from the outlet line to the inlet line, wherein the recycle line is connected with an overhead condenser and downstream thereof with a gas-liquid separator, wherein the gas-liquid separator is connected with a liquid line and is connected with a gas line, wherein the liquid line leads into the bottom dividing-wall distillation column.

7. The method in accordance with any of claims 3 to 6, wherein the sidewall of the bottom dividing-wall distillation column comprises an outlet line and an inlet line, both being connected with a recycle line leading from the outlet line to the inlet line, wherein the recycle line is connected with a side condenser and downstream thereof with a gas-liquid separator, wherein the gas-liquid separator is connected with a liquid line and is connected with a gas line, wherein the liquid line leads into the bottom dividing-wall distillation column.

8. The method in accordance with any of the preceding claims, wherein the at least one melt crystallization step b) comprises at least one melt crystallization stage being selected from the group consisting of falling film crystallization stage, static crystallization stage and suspension crystallization stage, and preferably comprises at least one falling film crystallization stage.

9. The method in accordance with any of the preceding claims, wherein step b) comprises one to five, preferably one to four, more preferably one to three and most preferably one to two falling film crystallization stages.

10. The method in accordance with claim 9, wherein the pre-purified composition obtained in step a) and subjected in step b) to at least one melt crystallization step is fed into a first of the two to five falling film crystallization stages so as to produce a first ethylene carbonate enriched crystallized fraction and a residue fraction, wherein the first ethylene carbonate enriched crystallized fraction is fed into a second of the two to five falling film crystallization stages, wherein in any of the second and of the optional third to fifth falling film crystallization stages an ethylene carbonate enriched crystallized fraction and a residue fraction is produced, wherein each of the ethylene carbonate enriched crystallized fractions produced in the second and the optional third to fourth falling film crystallization stages is fed into a downstream ethylene carbonate crystallization stage and each of the residue fractions produced in the second and the optional third to five falling film crystallization stages is fed into an upstream falling film crystallization stage, wherein the ethylene carbonate enriched crystallized fraction of the most downstream of the falling film crystallization stages is the purified ethylene carbonate composition.

11. The method in accordance with claim 10, wherein producing an ethylene carbonate enriched crystallized fraction and a residue fraction in a crystallization stage comprises the steps of removing the remaining liquid from the crystallization stage as residue fraction after termination of the crystallization in the crystallization stage, of melting the crystal layer obtained in the crystallization stage and of withdrawing the obtained crystal melt as ethylene carbonate enriched crystallized fraction from the crystallization stage, wherein before melting the crystal layer obtained in the crystallization stage, one or more sweating steps of the crystal layer are carried out so as to obtain one or more sweating fractions and a purified crystal layer.

12. The method in accordance with any of the preceding claims, wherein the purified ethylene carbonate composition comprises 50 ppm or less and preferably 10 ppm or less impurities.

13. A plant for purifying ethylene carbonate from a crude ethylene carbonate composition, wherein the plant comprises at least one distillation column and at least one crystallizer, wherein the at least one distillation column is a bottom dividing-wall distillation column, which comprises a dividing wall being connected with and extending from the bottom of the distillation column at least substantially vertically upwards over a part of the height of the distillation column thereby subdividing the bottom section of the dividing-wall distillation column, seen in cross-section of the dividing-wall distillation column, into a first bottom subsection and into a second bottom subsection, wherein the bottom dividing-wall distillation column further comprises an inlet line for a crude composition containing ethylene carbonate, an overhead outlet line, a bottom outlet line and a side outlet line, wherein the at least one crystallizer comprises an inlet line for a pre-purified composition and an outlet line for purified ethylene carbonate composition, wherein the side outlet line of the bottom dividing-wall distillation column is connected with the inlet line for pre-purified composition of the at least one crystallizer.

14. The plant in accordance with claim 13, wherein the bottom dividing-wall distillation column further comprises a partition wall comprising a first portion extending at least essentially vertically downwards and a second portion being connected with an upper part of the first portion of the partition wall and extending to the inner wall of the bottom dividing-wall distillation column, wherein the first portion of the partition wall extends above and/or at least partially within the second bottom subsection, wherein the inlet line being connected with the reboiler being connected with the outlet line of the second bottom subsection is connected with the wall of the bottom dividing-wall distillation column at a location being below the second portion of the partition wall and between the upper and lower ends of the first portion of the partition wall.

15. The plant in accordance with claim 13 or 14, wherein the bottom dividing-wall distillation column comprises a side fraction outlet and further comprises a separation wall comprising a first portion extending at least essentially vertically downwards and a second portion being connected with an upper part of the first portion of the separation wall and extending to the inner wall of the bottom dividing-wall distillation column, wherein the first portion of the separation wall extends above and/or at least partially within the first bottom subsection, wherein the inlet line being connected with the reboiler being connected with the outlet line of the first bottom subsection is connected with the bottom dividing-wall distillation column at a location being below the second portion of the separation wall and between the upper and lower ends of the first portion of the separation wall, wherein the uppermost point of the second portion of the separation wall is located below the side fraction outlet.
